Europäisches Patentamt

European Patent Office

Office européen des brevets

(11) Veröffentlichungsnummer: **0 356 892 B1**

(12) **EUROPÄISCHE PATENTSCHRIFT**

(45) Veröffentlichungstag der Patentschrift: **26.01.94**

(21) Anmeldenummer: **89115509.5**

(22) Anmeldetag: **23.08.89**

(51) Int. Cl.⁵: **C07C 22/00**, C07C 17/20

(54) **Verfahren zur Herstellung von teilfluorierten Ethanen.**

(30) Priorität: **27.08.88 DE 3829098**

(43) Veröffentlichungstag der Anmeldung:
**07.03.90 Patentblatt 90/10**

(45) Bekanntmachung des Hinweises auf die
Patenterteilung:
**26.01.94 Patentblatt 94/04**

(84) Benannte Vertragsstaaten:
**DE ES FR GB GR IT NL**

(56) Entgegenhaltungen:
**EP-A- 0 130 532**
**US-A- 4 145 368**

(73) Patentinhaber: **HOECHST AKTIENGESELL-SCHAFT**

**D-65926 Frankfurt(DE)**

(72) Erfinder: **Cremer, Hans Robert, Dr.**
**Pappelstrasse 1**
**D-5014 Kerpen 4(DE)**
Erfinder: **Noichl, Harald, Dr.**
**Am Flachsland 52**
**D-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

EP 0 356 892 B1

**Beschreibung**

Die Erfindung betrifft ein Verfahren zur Herstellung von teilfluorierten Ethanen der allgemeinen Formel

$$CF_3\text{-}CFH_xCl_{2-x}$$

mit x = 0, 1, 2.

Teilfluorierte Ethane der allgemeinen Formel $CF_3\text{-}CFH_xCl_{2-x}$ haben wünschenswerte Eigenschaften (chemisch inert, schwer entflammbar, nicht toxisch und nicht materialagressiv) der perhalogenierten Fluorchlorkohlenwasserstoffe. Die perhalogenierten Fluorchlorkohlenwasserstoffe werden für den Abbau der oberen Ozonschicht, die die Erdoberfläche vor einer intensiven UV-Strahlung schützt, verantwortlich. gemacht. Teilfluorierte Ethane der allgemeinen Formel $CF_3\text{-}CFH_xCl_{2-x}$ (x = 0, 1, 2) sind entweder chlorfrei oder werden bereits zum großen Teil in den unteren Atmosphärenschichten abgebaut und haben deshalb nur ein geringes Gefährdungspotential für die obere Ozonschicht

Es besteht daher ein erhebliches Interesse an teilfluorierten Ethanen als Austauschstoff, der die obere Ozonschicht nicht zerstört, anstelle perhalogenierter Fluorchlorkohlenwasserstoffe.

Teilfluorierte Ethane werden gemäß der US-A-2,748,177 durch Umsetzung von $CCl_4$, $CCl_3\text{-}CClF_2$, $CClF_2\text{-}CF_3$ oder $CCl_2F\text{-}CClF_2$ mit wasserfreiem Fluorwasserstoff an einem Aluminiumfluorid-Katalysator bei Temperaturen von 175 bis 450 °C hergestellt. Nachteilig bei diesem Verfahren ist, neben der schwierigen Handhabung von Fluorwasserstoff, die Bildung konstitutionsisomerer Gemische von teilfluorierten Ethanen, die teilweise nur unter aufwendigen Destillationsbedingungen aufgetrennt werden könne. Hinzu kommt, daß in erheblichem Maß wirtschaftlich nicht verwertbare Fluorchlorethane bei dieser Arbeitsweise anfallen. Nach US-A-4 145 368 entsteht aus $CF_3\text{-}CCl_3$ und $CF_3\text{-}CH_2Cl$ an einem Chrom-Katalysator $CF_3\text{-}CCl_2H$, eine Fluorierung tritt nicht ein. Es bestand die Aufgabe, ein Verfahren zur Herstellung von teilfluorierten Ethanen der allgemeinen Formel

$$CF_3\text{-}CFH_xCl_{2-x}$$

mit x = 0, 1, 2 bereitzustellen, welches mit hoher Selektivität die gezielte Herstellung dieser Verbindungen erlaubt und die ohne großen Destillationsaufwand als Reinprodukt isolierbar sind.

Gegenstand der Erfindung ist ein Verfahren zur Herstellung von teilfluorierten Ethanen der allgemeinen Formel

$$CF_3\text{-}CFH_xCl_{2-x}$$

mit x = 0, 1, 2, dadurch gekennzeichnet, daß man als Reaktant teilfluoriertes Ethan der allgemeinen Formel

$$C_2F_3H_xCl_{3-x}$$

mit x = 0, 1, 2
und als Fluorierungsmittel teilfluorierte Ethane der allgemeinen Formel

$$CF_{3-x}Cl_x\text{-}CH_{3-y}Cl_y$$

mit x = 0, 1, 2 und y = 0, 1, 2, 3
in Gegenwart eines Chrom-Katalysators bei Temperaturen von 150 bis 600 °C gasförmig miteinander umsetzt, wobei ausgeschlossen wird, daß der Reaktant und das Fluorierungsmittel dieselbe chemische Strukturformel haben, und wobei im Falle des Reaktanten $CF_3\text{-}CCl_3$ und des Fluorierungsmittels $CF_3\text{-}CH_2Cl$ der Reaktant im Überschuß eingesetzt wird. Bei Umsetzungen, bei denen Reaktant und Fluorierungsmittel identisch sind, kommt es beim gasförmigen Überleiten dieser Verbindung über den Chrom-Katalysator zu einer Dismutierungsreaktion, bei der nur ein sehr geringer Umsatz zu einer Vielzahl von Verbindungen erfolgt, wie im nachfolgenden Vergleichsbeispiel 1 beschrieben ist.

Es hat sich gezeigt, daß die gewünschten teilfluorierten Ethane in besonders hoher Selektivität erhalten werden, wenn man als Reaktant teilfluoriertes Ethan der allgemeinen Formel

$$CF_3\text{-}CH_xCl_{3-x}$$

mit x = 0, 1, 2 einsetzt, was daher bevorzugt ist.

2

Für das erfindungsgemäße Verfahren benutzt man als Chrom-Katalysator Chrom(III)-Verbindungen wie Oxide, Hydroxide, Nitrate oder Halogenide. Vorzugsweise werden die Chrom-(III)-Verbindungen vor ihrem Einsatz zur Umfluorierung mit gasförmigem Fluorwasserstoff aktiviert.

Besonders hohe Selektivitäten werden erreicht, wenn man den Chrom-Katalysator zusammen mit einem Träger wie Magnesiumoxid oder Graphit einsetzt und den Chrom-Träger-Katalysator durch eine Fluorwasserstoff-Behandlung aktiviert. Eine Möglichkeit für die Herstellung des Chrom-Träger-Katalysators ist in der US-A-4,547,483 beschrieben.

Dieser Chrom-Träger-Katalysator besteht aus mindestens 55 Gew.-% Magnesiumfluorid und 0,5 bis 29 Gew.-% Chrom und hat ein Atomverhältnis Magnesium : Chrom = 1,5 bis 50. In diesem Katalysator liegt das Chrom als Chromoxifluorid vor.

Zur Herstellung des ebengenannten Chrom-Träger-Katalysators wird 1 Mol eines wasserlöslichen Chrom(III)-Salzes mit mindestens 1,5 Mol Magnesiumhydroxid oder Magnesiumoxid in Gegenwart von Wasser umgesetzt, und so,gegebenenfalls durch Eindampfen,die Reaktionsmischung in einen Teig, der Chromhydroxid und ein Magnesiumsalz enthält, überführt. Der Teig wird getrocknet und bei Temperaturen von 20 bis 500 °C mit Fluorwasserstoff behandelt. Der Teig kann ferner in Abhängigkeit von dem angewandten Magnesiumüberschuß noch MgO bzw. Mg(OH)$_2$ enthalten.

Die Behandlung mit Fluorwasserstoff erfolgt zweckmäßigerweise bei einer Temperatur von 100 bis 400 °C. Pro Mol eingesetzter Metallverbindung (Chromsalz, Magnesiumoxid) wird der Katalysator mit mindestens 2 Mol Fluorwasserstoff behandelt. Die Fluorierungszeit beträgt 0,5 bis 10 Stunden. Um das entstehende Wasser schneller zu entfernen und unerwünschte Temperaturspitzen zu vermeiden, kann man HF durch ein Inertgas (z.B. N$_2$ oder Luft) verdünnen.

Man kann auch Katalysatoren nahezu gleicher Bruttozusammensetzung herstellen, indem man pulverförmiges Chromoxidhydrat mit pulverförmigen Magnesiumfluorid vermischt und mit Fluorwasserstoff behandelt.

Eine hohe Selektivität des erfindungsgemäßen Verfahren wird erreicht, wenn man es bei einer Temperatur von 250 bis 400 °C, insbesondere von 320 bis 360 °C durchführt. Das erfindungsgemäße Verfahren arbeitet gleichermaßen gut bei Unter-, Normal-, oder Überdruck.

Zur Erzielung hoher Umsätz soll man eine mittlere Kontaktzeit von 1 bis 300 Sekunden, insbesondere von 10 bis 100 Sekunden, bei der Umsetzung einhalten. Die mittlere Kontaktzeit wird berechnet nach der Formel:

$$\text{Mittlere Kontaktzeit [S]} = \frac{\text{Volumen des Katalysators [ml]}}{\substack{\text{Volumen der zugeführten}\\ \text{Einsatzstoffe pro Sekunde [ml/s]}}}$$

Besonders hohe Selektivitäten werden erreicht, wenn man ein molares Verhältnis Reaktant : Fluorierungsmittel = (1 bis 15) : (10 bis 1), vorzugsweise (1 bis 7) : (5 bis 1) einstellt.

Es wurde weiter gefunden, daß ein Überschuß an Reaktant die Selektivität der Reaktion günstig beeinflußt.

Das erfindungsgemäße Verfahren liefert auch dann gute Ergebnisse, wenn man als Fluorierungsmittel ein Gemisch teilfluorierter Ethane der allgemeinen Formel

$CF_{3-x}Cl_x\text{-}CH_{3-y}Cl_y$

mit x = 0, 1, 2 und y = 0, 1, 2, 3 einsetzt.

3

In der Tabelle 1 sind Gleichungen möglicher erfindungsgemäßer Reaktionen aufgelistet:

## Tabelle 1

| Reaktant | Fluorierungsmittel | Endprodukt | Nebenprodukt |
|---|---|---|---|
| $3\ CF_3\text{-}CCl_3$ + | $CF_3\text{-}CH_2Cl$ | $\rightarrow$ $3\ CF_3\text{-}CFCl_2$ + | $CCl_2\text{=}CHCl$ + $HCl$ |
| $2\ CF_3\text{-}CCl_3$ + | $CClF_2\text{-}CH_2Cl$ | $\rightarrow$ $2\ CF_3\text{-}CFCl_2$ + | $CCl_2\text{=}CHCl$ + $HCl$ |
| $CF_3\text{-}CCl_3$ + | $CCl_2F\text{-}CH_2Cl$ | $\rightarrow$ $CF_3\text{-}CFCl_2$ + | $CCl_2\text{=}CHCl$ + $HCl$ |
| $3\ CF_3\text{-}CCl_3$ + | $CF_3\text{-}CHCl_2$ | $\rightarrow$ $3\ CF_3\text{-}CFCl_2$ + | $CCl_2\text{=}CCl_2$ + $HCl$ |
| $2\ CF_3\text{-}CCl_3$ + | $CF_3\text{-}CHCl_2$ | $\rightarrow$ $2\ CF_3\text{-}CFCl_2$ + | $CCl_2\text{=}CClF$ + $HCl$ |
| $2\ CF_3\text{-}CCl_3$ + | $CClF_2\text{-}CHCl_2$ | $\rightarrow$ $2\ CF_3\text{-}CFCl_2$ + | $CCl_2\text{=}CCl_2$ + $HCl$ |
| $CF_3\text{-}CCl_3$ + | $CClF_2\text{-}CHCl_2$ | $\rightarrow$ $CF_3\text{-}CFCl_2$ + | $CCl_2\text{=}CClF$ + $HCl$ |
| $CF_3\text{-}CCl_3$ + | $CCl_2F\text{-}CHCl_2$ | $\rightarrow$ $CF_3\text{-}CFCl_2$ + | $CCl_2\text{=}CCl_2$ + $HCl$ |
| $2\ CF_3\text{-}CCl_3$ + | $CF_3\text{-}CH_3$ | $\rightarrow$ $2\ CF_3\text{-}CFCl_2$ + | $CCl_2\text{=}CH_2$ + $HF$ |
| $CF_3\text{-}CCl_3$ + | $CClF_2\text{-}CH_3$ | $\rightarrow$ $CF_3\text{-}CFCl_2$ + | $CCl_2\text{=}CH_2$ + $HF$ |
| $CF_3\text{-}CCl_3$ + | $CCl_2F\text{-}CH_3$ | $\rightarrow$ $CF_3\text{-}CFCl_2$ + | $CCl_2\text{=}CH_2$ + $HCl$ |
| $CF_3\text{-}CCl_3$ + | $CCl_2F\text{-}CCl_3$ | $\rightarrow$ $CF_3\text{-}CFCl_2$ + | $CCl_2\text{=}CCl_2$ + $Cl_2$ |
| $2\ CF_3\text{-}CHCl_2$ + | $CF_3\text{-}CH_2Cl$ | $\rightarrow$ $2\ CF_3\text{-}CHFCl$ + | $CCl_2\text{=}CHCl$ + $HF$ |
| $CF_3\text{-}CHCl_2$ + | $CClF_2\text{-}CH_2Cl$ | $\rightarrow$ $CF_3\text{-}CHFCl$ + | $CCl_2\text{=}CHCl$ + $HF$ |
| $CF_3\text{-}CH_2Cl$ + | $CClF_2\text{-}CH_2Cl$ | $\rightarrow$ $CF_3\text{-}CH_2F$ + | $CCl_2\text{=}CHCl$ + $HF$ |
| $2\ CF_3\text{-}CHCl_2$ + | $CF_3\text{-}CH_3$ | $\rightarrow$ $2\ CF_3\text{-}CHFCl$ + | $CCl_2\text{=}CH_2$ + $HF$ |
| $CF_3\text{-}CHCl_2$ + | $CClF_2\text{-}CH_3$ | $\rightarrow$ $CF_3\text{-}CHFCl$ + | $CCl_2\text{=}CH_2$ + $HF$ |
| $2\ CF_3\text{-}CH_2Cl$ + | $CF_3\text{-}CH_3$ | $\rightarrow$ $2\ CF_3\text{-}CH_2F$ + | $CCl_2\text{=}CH_2$ + $HF$ |
| $CF_3\text{-}CH_2Cl$ + | $CClF_2\text{-}CH_3$ | $\rightarrow$ $CF_3\text{-}CH_2F$ + | $CCl_2\text{=}CH_2$ + $HF$ |

Mit den folgenden, in Tabelle 2 aufgelisteten Fluorierungsmitteln einzeln oder im Gemisch miteinander werden besonders hohe Umsätze erzielt

Tabelle 2

| Fluorierungsmittel | Formel |
|---|---|
| 1,1-Dichlor-1-fluorethan | $CCl_2F\text{-}CH_3$ |
| 1-Chlor-1,1-difluorethan | $CClF_2\text{-}CH_3$ |
| 1,1,1-Trifluorethan | $CF_3\text{-}CH_3$ |
| 1-Fluor-1,1,2-trichlorethan | $CCl_2F\text{-}CH_2Cl$ |
| 1,2-Dichlor-1,1-difluorethan | $CClF_2\text{-}CH_2Cl$ |
| 1-Chlor-2,2,2-trifluorethan | $CF_3\text{-}CH_2Cl$ |
| 1-Fluor-1,1,2,2-tetrachlorethan | $CCl_2F\text{-}CHCl_2$ |
| 1,1-Difluor-1,2,2-trichlorethan | $CClF_2\text{-}CHCl_2$ |
| 1,1-Dichlor-2,2,2-trifluorethan | $CF_3\text{-}CHCl_2$ |
| Fluorpentachlorethan | $CCl_2F\text{-}CCl_3$ |
| 1,1-Difluor-tetrachlorethan | $CClF_2\text{-}CCl_3$ |

Von Vorteil ist es, daß die gewünschten teilfluorierten Ethane aufgrund ihres niedrigen Siedepunktes leicht aus dem Umsetzungsprodukt durch eine fraktionierte Destillation in reiner Form isoliert werden können.

Wird das Fluorierungsmittel im Überschuß eingesetzt, kann das überschüssige Fluorierungsmittel zusammen mit den gebildeten Nebenprodukten erneut als Fluorierungsmittel verwendet werden oder die gebildeten Nebenprodukte werden in einer nachgeschalteten Fluorierungsstufe mit Fluorwasserstoff regeneriert und als Fluorierungsmittel wieder eingesetzt.

Eine geeignete Ausführungsform des erfindungsgemäßen Verfahren ist die folgende: Zunächst werden der Reaktant und das Fluorierungsmittel verdampft und in einer Mischstrecke gasförmig gemischt und auf die Umsetzungstemperatur erwärmt. Diese Gasmischung wird in die Kontaktzone eingeschleust. Die Kontaktzone besteht aus einem temperierten Rohr, in dem eine lose Katalysatorschüttung angeordnet ist. Die Umsetzungsprodukte werden kondensiert und die organische Phase fraktioniert destilliert.

Die Nomenklatur der Fluorkohlenwasserstoffe erfolgt nach der in der Technik gebräuchlichen Art, die erläutert ist in "Römpps Chemie-Lexikon", Frankh'sche Verlangshandlung, Stuttgart, (1973), Bd. 2, Seite 1172.

Die Erfindung wird anhand der folgenden Beispiele näher erläutert:

Die einzelnen Fraktionen wurden gaschromatographisch analysiert und zusätzlich mit der $^{19}$F- und $^1$H-NMR-Methode identifiziert. Die angegebenen Prozente sind Gewichtsprozente.

**Beispiel 1**

In ein elektrisch beheiztes Glasrohr von 50 cm Länge und 1 cm Innendurchmesser wurden 30 ml Chrom-Magnesium-Katalysator, hergestellt nach US-A-4,547,483, eingebracht. 1,1,1-Trichlor-trifluorethan (R113a) und 1-Chlor-2,2,2-trifluorethan (R133a) wurden verdampft, in eine 70 cm langen, temperierten Rohr vermischt und mit einem Molverhältnis von 3,5 : 1 über den Katalysator geleitet. Die mittlere Katalysatorkontaktzeit betrug 37 Sekunden. Der Versuch wurde bei verschiedenen Temperaturen jeweils 12 Stunden lang durchgeführt. Die Resultate sind in der Tabelle 3 angegeben.

## Tabelle 3

| Komponenten | Temperaturen (°C) | | | | |
|---|---|---|---|---|---|
| | 280 | 300 | 320 | 340 | 360 |
| $CF_3-CFCl_2$ (Endprodukt) | 16,4 % | 24,5 % | 42,9 % | 52,9 % | 58,4 % |
| $CF_3-CCl_3$ (Reaktant) | 66,2 % | 57,8 % | 39,0 % | 26,0 % | 19,7 % |
| $CF_3-CH_2Cl$ (Fluorierungs-mittel) | 11,8 % | 10,3 % | 5,1 % | 3,3 % | 1,1 % |

### Fortsetzung der Tabelle 3

| Komponenten | Temperaturen (°C) | | | | |
|---|---|---|---|---|---|
| | 280 | 300 | 320 | 340 | 360 |
| $CCl_2=CHCl$ (Nebenprodukt) | 4,6 % | 6,6 % | 11,8 % | 14,7 % | 16,3 % |
| andere Produkte | <1,0 % | <1,0 % | 1,2 % | 3,1 % | 4,5 % |
| Umsatz (bez. auf R133a): | 24,3 % | 35,6 % | 66,1 % | 78,8 % | 92,6 % |
| Umsatz (bez. auf R113a): | 21,4 % | 31,7 % | 54,7 % | 69,0 % | 76,5 % |

Eine Temperaturerhöhung am Katalysator führt zu einer Umsatzvergrößerung; es steigt allerdings auch der Anteil an anderen Produkten.

**Beispiel 2**

In der Versuchsanordnung von Beispiel 1 wurden bei 340 °C 1,1 1-Trichlor-trifluorethan (R113a) und 1-Chlor-2,2,2-trifluorethan (R133a) im Molverhältnis von 0,6 : 1 über den Chrom-Magnesium-Katalysator (wie in Beispiel 1) geleitet. Die mittlere Katalysatorverweilzeit betrug 21 Sekunden. Der Versuch wurde 10 Stunden lang durchgeführt. Das Resultat war wie folgt:

| | |
|---|---|
| $CF_3$-$CFCl_2$ | 14,2 % |
| $CF_3$-$CCl_3$ | 32,3 % |
| $CF_3$-$CH_2Cl$ | 45,6 % |
| $CCl_2 = CHCl$ | 4,0 % |
| $CF_3$-$CHCl_2$ | < 0,2 % |
| andere Produkte | 3,7 % |
| Umsatz (bez. auf R113a) | 32,7 % |

Der Vergleich der Beispiele 2 und 1 (340°) zeigt, daß ein höherer Reaktantenanteil im Gasgemisch zu einer Selektivitätserhöhung führt.

**Beispiel 3**

In der Versuchsanordnung von Beispiel 1 wurden 30 ml Chromoxid-fluorid-Katalysator gegeben, welcher zuvor aus Chromoxidhydrat (Guignet Grün) nach der in der Patentschrift US-A-4,145,368 beschriebenen Methode mit $HF/N_2$ hergestellt wurde. Bei 340 °C wurden 1,1,1-Trichlortrifluorethan(R113a) und 1-Chlor-2,2,2-trifluorethan (R133a) über den Katalysator geleitet. Die Molverhältnisse, die Kontaktzeiten sowie die Anteile der organischen Komponenten sind in der Tabelle 4 zusammengefaßt. Der Versuch wurde 12 Stunden lang durchgeführt.

**Tabelle 4**

| Molverhältnis R113a : R133a | 0,6 : 1 | 3,5 : 1 |
|---|---|---|
| Mittlere Kontaktzeit: (Sekunden) | 21 | 37 |
| Komponenten: | | |
| $CF_3$-$CFCl_2$ (Endprodukt) | 8,9 % | 32,3 % |
| $CF_3$-$CCl_3$ (Reaktant) | 37,9 % | 46,9 % |
| $CF_3$-$CH_2Cl$ (Fluorierungsmittel) | 46,3 % | 7,4 % |
| $CCl_2$=CHCl (Nebenprodukt) | 2,6 % | 9,2 % |
| $CF_3$-$CHCl_2$ (Nebenprodukt) | 1,3 % | 1,2 % |
| andere Produkte | 3,1 % | 3,0 % |
| Umsatz bez. auf R113a: | 21,7 % | 43,1 % |
| Umsatz bez. auf R133a: | | 52,7 % |

Das Beispiel 3 zeigt den Einsatz eines anderen Katalysators als in den Beispielen 1 und 2.

6

**Beispiel 4**

In der Versuchsanordnung von Beispiel 1, gefüllt mit Chrom-Magnesium-Katalysatorwie in Beispiel 1, wurden bei einer Temperatur von 340 °C 1,1,1-Trichlor-trifluorethan (R113a) und 1,1-Difluor-1,2,2-trichlorethan (R122) im Molverhältnis von 3,4 : 1 umgesetzt. Die mittlere Katalysatorkontaktzeit betrug 42 Sekunden. Der Versuch wurde 12 Stunden lang durchgeführt. Das Resultat war wie folgt:

| | |
|---|---|
| $CF_3$-$CFCl_2$ (Endprodukt) | 45,8 % |
| $CF_3$-$CCl_3$ (Reaktant) | 30,0 % |
| $CF_2Cl$-$CHCl_2$ (Fluorierungsmittel) | 0,8 % |
| $CCl_2 = CCl_2$ (Nebenprodukt) | 12,1 % |
| $CCl_2 = CClF$ ( " ) | 8,3 % |
| andere Produkte | 3,8 % |

| Umsatz | |
|---|---|
| bez. auf R122 | 96,5 % |
| bez. auf R113a | 62,6 % |

**Beispiel 5**

In der Versuchsanordnung von Beispiel 1 wurde bei einer Temperatur von 340 °C ein Gemisch aus 1,1,1-Trichlortrifluorethan (R113a) und 1,2-Dichlor-1,1-difluorethan (R132b) in einem Molverhältnis von 4,1 : 1 über den im Beispiel 1 beschriebenen Katalysator geleitet. Die mittlere Katalysatorkontaktzeit betrug 42 Sekunden. Der Versuch wurde 12 Stunden lang durchgeführt. Das Resultat war wie folgt:

| | |
|---|---|
| $CF_3$-$CFCl_2$ (Endprodukt) | 38,1 % |
| $CF_3$-$CCl_3$ (Reaktant) | 44,6 % |
| $CF_2Cl$-$CH_2Cl$ (Fluorierungsmittel) | < 0,1 % |
| $CCl_2 = CHCl$ (Nebenprodukt) | 14,3 % |
| andere Produkte | 2,9 % |

| Umsatz | |
|---|---|
| bez. auf R132b | >99,9 % |
| bez. auf R113a | 48,4 % |

Die Beispiele 4 und 5 unterscheiden sich von den voranstehenden Beispielen 1 bis 3 durch die Wahl des Fluorierungsmittels.

**Beispiel 6**

In der Versuchsanordnung von Beispiel 1 wurden bei 360 °C verschiedene Mischungen aus $CF_3$-$CHCl_2$ (R123) und $CF_3$-$CH_2Cl$ (R133a) über den in Beispiel 1 beschriebenen Chrom-Magnesium-Katalysator geleitet.
Die mittleren Katalysatorkontaktzeiten, Molverhältnisse und Anteile der organischen Komponenten sind in Tabelle 6 zusammengestellt.
Der Versuch wurde 12 Stunden lang durchgeführt.

## Tabelle 6

| Molverhältnis R123 :R133a: | 0,8:1 | 1,8:1 | 2,6:1 | 4,4:1 |
|---|---|---|---|---|
| Mittlere Kontaktzeit (Sekunden) | 18 | 19 | 23 | 22 |
| **Komponenten:** | | | | |
| $CF_3$-CFHCl (Endprodukt) | 18,0 % | 15,4 % | 21,2 % | 19,1 % |
| $CF_3$-$CH_2$Cl (Fluorierungsmittel) | 37,6 % | 22,7 % | 9,9 % | 5,6 % |
| $CF_3$-$CHCl_2$ (Reaktant) | 27,6 % | 52,5 % | 48,7 % | 60,7 % |
| $CCl_2$=CHCl (Nebenprodukt) | 11,5 % | 7,4 % | 12,7 % | 9,7 % |
| andere Produkte | 6,6 % | 5,9 % | 7,1 % | 4,9 % |
| **Umsatz** | | | | |
| bez. auf R123: | 42,3 % | 24,7 % | 32,7 % | 26,1 % |
| bez. auf R133a: | | | 48,2 % | 59,3 % |

**Beispiel 7**

In den im Beispiel 1 beschriebenen Reaktor wurden 30 ml des dort beschriebenen Chrom-Magnesium-Katalysators eingebracht. Bei 360 °C wurden 1,1-Dichlor-2,2,2-trifluorethan (R123) und 1,1,1-Trifluorethan (R143a) in einem Molverhältnis von 2,6 : 1 zudosiert.

Die Katalysatorkontaktzeit betrug 29 Sekunden. Der Versuch wurde 12 Stunden lang durchgeführt. Die gaschromatographische Analyse ergab für den organischen Produktanteil folgende Zusammensetzung:

| | |
|---|---|
| $CF_3$-CHFCl (Endprodukt) | 15,0 % |
| $CF_3$-$CH_3$ (Fluorierungsmittel) | 13,3 % |
| $CF_3$-$CHCl_2$ (Reaktant) | 65,5 % |
| $CCl_2$ = $CH_2$ (Nebenprodukt) | 4,9 % |
| andere Produkte | 1,3 % |

| Umsatz | |
|---|---|
| bez. auf R143a | 25,8 % |
| bez. auf R123 | 20,4 % |

**Beispiel 8**

In der Versuchsanordnung aus Beispiel 1 wurden bei 400 °C 1-Chlor-2,2,2-trifluorethan (R133a) und 1,1,1-Trifluorethan (R143a) im Verhältnis von 2,2 : 1 über den Chrom-Magnesium-Katalysator geleitet. Die Katalysatorkontaktzeit belief sich auf 29 Sekunden. Der Versuch wurde 12 Stunden lang durchgeführt. Die gaschromatographische Untersuchung des organischen Teils des Rohproduktes ergab folgende Zusammensetzung:

| CF$_3$-CH$_2$F (Endprodukt) | 7,9 % |
|---|---|
| CF$_3$-CH$_3$ (Fluorierungsmittel) | 23,1 % |
| CF$_3$-CH$_2$Cl (Reaktant) | 60,5 % |
| andere Produkte (CCl$_2$ = CH$_2$, CF$_2$ = CHCl) | 8,5 % |

| Umsatz | |
|---|---|
| bez. auf R143a | 12,3 % |
| bez. auf R133a | 11,1 % |

Die Beispiele 6 bis 8 belegen den Erfindungsgegenstand für weitere Reaktant-Fluorierungsmittel-Umsetzungen.

**Vergleichsbeispiel 1**

Beispiel für die Dismutierung.

Das Beispiel 1 wurde wiederholt mit der Änderung, daß 1,1,1-Trichlor-trifluorethan ohne Fluorierungsmittel über den Chrom-Magnesium-Katalysator des Beispiels 1 bei 340 °C geleitet wurde. Die mittlere Katalysatorkontaktzeit betrug 37 Sekunden. Der Versuch wurde 12 Stunden lang durchgeführt. Das Resultat war wie folgt:

| CF$_3$-CFCl$_2$ (Endprodukt) | 1,2 % |
|---|---|
| CF$_3$-CCl$_3$ (Reaktant) | 97,7 % |
| andere Produkte | 1,1 % |

**Vergleichsbeispiel 2**

Das Beispiel 1 wurde wiederholt, wobei jetzt als Katalysator Aluminium-fluorid untersucht wurde. Der Katalysator wurde aus aktiviertem Aluminium-oxid (Fa. Harshaw/Filtrol, Cleveland, Ohio) nach der US-A-3,087,974 mit CCl$_2$F-CClF$_2$ (R113) dargestellt. Bei 340 °C wurden dann verschiedene Mischungen aus CF$_3$-CCl$_3$ (R113a) und CF$_3$-CH$_2$Cl (R133a) über den Katalysator geleitet. Die mittleren Kontaktzeiten, Molverhältnisse und Anteile der organischen Komponenten sind in Tabelle 5 notiert. Der Versuch wurde 12 Stunden lang durchgeführt.

EP 0 356 892 B1

## Tabelle 5

| Molverhältnis R 113a : R133a: | 0,5 : 1 | 3,7 : 1 |
|---|---|---|

| | | |
|---|---|---|
| Mittlere Kontaktzeit: (Sekunden) | 21 | 47 |
| | | |
| Komponenten: | | |
| $CF_3$-$CFCl_2$ (Endprodukt) | 0,3 % | 0,7 % |
| $CF_3$-$CCl_3$ (Reaktant) | 39,3 % | 82,2 % |
| $CF_3$-$CH_2Cl$ (Fluorierungsmittel) | 53,9 % | 14,1 % |
| $CF_2$=CHCl (Nebenprodukt) | 6,1 % | 2,2 % |
| $CF_3$-$CHCl_2$ (Nebenprodukt) | 0,4 % | 0,7 % |
| | | |
| Umsatz bez. auf R113a: | < 1,0 % | 0,5 % |

Das Vergleichsbeispiel 2 belegt, daß Aluminiumfluorid-Katalysatoren als Umfluorierungs-Katalysator unbrauchbar sind.

**Patentansprüche**

1. Verfahren zur Herstellung von teilfluorierten Ethanen der allgemeinen Formel

   $$CF_3\text{-}CFH_xCl_{2-x}$$

   mit x = 0, 1, 2,
   dadurch gekennzeichnet, daß man als Reaktant teilfluoriertes Ethan der allgemeinen Formel

   $$C_2F_3H_xCl_{3-x}$$

   mit x = 0, 1, 2
   und als Fluorierungsmittel teilfluorierte Ethane der allgemeinen Formel

   $$CF_{3-x}Cl_x\text{-}CH_{3-y}Cl_y$$

   mit x = 0, 1, 2 und y = 0, 1, 2, 3
   in Gegenwart eines Chrom-Katalysators bei Temperaturen von 150 bis 600 °C gasförmig miteinander umsetzt, wobei ausgeschlossen wird, daß der Reaktant und das Fluorierungsmittel dieselbe chemische Strukturformel haben, und wobei im Falle des Reaktanten $CF_3$-$CCl_3$ und des Fluorierungsmittels $CF_3$-$CH_2Cl$ der Reaktant im Überschuß eingesetzt wird.

2. Verfahren nach Anspruch 1, dadurch gekennzeichnet, daß man als Reaktant teilfluoriertes Ethan der allgemeinen Formel

   $$CF_3\text{-}CH_xCl_{3-x}$$

   mit x = 0, 1, 2 einsetzt.

3. Verfahren nach Anspruch 1 oder 2, dadurch gekennzeichnet, daß man als Chrom-Katalysator Chrom-(III)-Verbindungen einsetzt.

10

**4.** Verfahren nach einem der voranstehenden Ansprüche 1 bis 3, dadurch gekennzeichnet, daß man den Chrom-Katalysator zusammen mit einem Träger verwendet.

**5.** Verfahren nach Anspruch 3 oder 4, dadurch gekennzeichnet, daß man den Chrom-Katalysator durch eine Fluorwasserstoff-Behandlung aktiviert.

**6.** Verfahren nach einem der voranstehenden Ansprüche 1 bis 5, dadurch gekennzeichnet, daß man die Umsetzung bei einer Temperatur von 250 bis 400 °C durchführt.

**7.** Verfahren nach einem der voranstehenden Ansprüche 1 bis 6, dadurch gekennzeichnet, daß man die Umsetzung bei einer Temperatur von 320 bis 360 °C durchführt.

**8.** Verfahren nach einem der voranstehenden Ansprüche 1 bis 7, dadurch gekennzeichnet, daß man eine mittlere Kontaktzeit von 1 bis 300 Sekunden bei der Umsetzung einhält.

**9.** Verfahren nach einem der voranstehenden Ansprüche 1 bis 8, dadurch gekennzeichnet, daß man eine mittlere Kontaktzeit von 10 bis 100 Sekunden bei der Umsetzung einhält.

**10.** Verfahren nach einem der voranstehenden Ansprüche 1 bis 9, dadurch gekennzeichnet, daß man ein molares Verhältnis Reaktant : Fluorierungsmittel = (1 bis 15) : (10 bis 1) einstellt.

**11.** Verfahren nach einem der voranstehenden Ansprüche 1 bis 10, dadurch gekennzeichnet, daß man ein molares Verhältnis Reaktant : Fluorierungsmittel = (1 bis 7) : (5 bis 1) einstellt.

**12.** Verfahren nach einem der voranstehenden Ansprüche 1 bis 11, dadurch gekennzeichnet, daß man als Fluorierungsmittel ein Gemisch teilfluorierter Ethane der allgemeinen Formel

$$CF_{3-x}Cl_x\text{-}CH_{3-y}Cl_y$$

mit x = 0, 1, 2 und y = 0, 1, 2, 3 einsetzt.

## Claims

**1.** A process for the preparation of a partly fluorinated ethane of the formula

$$CF_3\text{-}CFH_xCl_{2-x}$$

in which x = 0, 1 or 2,
which comprises reacting, as the reactant, a partly fluorinated ethane of the formula

$$C_2F_3H_xCl_{3-x}$$

in which x = 0, 1 or 2
and, as the fluorinating agent, a partly fluorinated ethane of the formula

$$CF_{3-x}Cl_x\text{-}CH_{3-y}Cl_y$$

in which x = 0, 1 or 2 and y = 0, 1, 2 or 3
with one another in gaseous form in the presence of a chromium catalyst at temperatures of 150 to 600 °C, the reactant and the fluorinating agent having the same chemical structural formula being excluded and the reactant being employed in excess in the case where the reactant is $CF_3\text{-}CCl_3$ and the fluorinating agent is $CF_3\text{-}CH_2Cl$.

**2.** The process as claimed in claim 1, wherein a partly fluorinated ethane of the formula

$$CF_3\text{-}CH_xCl_{3-x}$$

in which x = 0, 1 or 2, is employed as the reactant.

11

3. The process as claimed in claim 1 or 2, wherein a chromium(III) compound is employed as the chromium catalyst.

4. The process as claimed in any one of the preceding claims 1 to 3, wherein the chromium catalyst is used together with a support.

5. The process as claimed in claim 3 or 4, wherein the chromium catalyst is activated by a hydrogen fluoride treatment.

6. The process as claimed in any one of the preceding claims 1 to 5, wherein the reaction is carried out at a temperature of 250 to 400 °C.

7. The process as claimed in any one of the preceding claims 1 to 6, wherein the reaction is carried out at a temperature of 320 to 360 °C.

8. The process as claimed in any one of the preceding claims 1 to 7, wherein an average contact time of 1 to 300 seconds is maintained during the reaction.

9. The process as claimed in any one of the preceding claims 1 to 8, wherein an average contact time of 10 to 100 seconds is maintained during the reaction.

10. The process as claimed in any one of the preceding claims 1 to 9, wherein a molar ratio of reactant:fluorinating agent = (1 to 15) : (10 to 1) is established.

11. The process as claimed in any one of the preceding claims 1 to 10, wherein a molar ratio of reactant:fluorinating agent = (1 to 7) : (5 to 1) is established.

12. The process as claimed in any one of the preceding claims 1 to 11, wherein a mixture of partly fluorinated ethanes of the formula

$$CF_{3-x}Cl_x\text{-}CH_{3-y}Cl_y$$

in which x = 0, 1 or 2 and y = 0, 1, 2 or 3, is employed as the fluorinating agent.

## Revendications

1. Procédé de préparation d'éthanes partiellement fluorés de formule générale :

$$CF_3\text{-}CFH_xCl_{2-x}$$

avec x = 0, 1, 2,
caractérisé en ce que l'on fait réagir l'un sur l'autre, sous forme gazeuse, comme réatif, de l'éthane partiellement fluoré de formule générale

$$C_2F_3H_xCl_{3-x}$$

avec x = 0, 1, 2
et, comme agent de fluoration, de l'éthane partiellement fluoré de formule générale

$$CF_{3-x}Cl_x\text{-}CH_{3-y}Cl_y$$

avec x = 0, 1, 2 et y = 0, 1, 2, 3
en présence d'un catalyseur au chrome à des températures de 150 à 600 °C, étant exclu que le réactif et l'agent de fluoration aient la même formule de structure chimique et dans le cas du réactif $CF_3$-$CCl_3$ et de l'agent de fluoration $CF_3$-$CH_2Cl$, le réactif étant utilisé en excès.

2. Procédé suivant la revendication 1, caractérisé en ce que l'on met en oeuvre comme réactif de l'éthane partiellement fluoré de formule générale

$$CF_3\text{-}CH_xCl_{3-x}$$

avec x = 0, 1, 2.

3. Procédé suivant la revendication 1 ou 2, caractérisé en ce que l'on met en oeuvre comme catalyseur au chrome des composés du chrome (III).

4. Procédé suivant l'une des revendications précédentes 1 à 3, caractérisé en ce que l'on utilise le catalyseur au chrome en association avec un support.

5. Procédé suivant la revendication 3 ou 4, caractérisé en ce que l'on active le catalyseur au chrome par un traitement avec le fluorure d'hydrogène.

6. Procédé suivant l'une des revendications précédentes 1 à 5, caractérisé en ce que l'on effectue la réaction à une température de 250 à 400°C.

7. Procédé suivant l'une des revendications précédentes 1 à 6, caractérisé en ce que l'on effectue la réaction à une température de 320 à 360°C.

8. Procédé suivant l'une des revendications précédentes 1 à 7, caractérisé en ce que l'on maintient un temps de contact moyen de 1 à 300 secondes lors de la réaction.

9. Procédé suivant l'une des revendications précédentes 1 à 8, caractérisé en ce que l'on maintient un temps de contact moyen de 10 à 100 secondes lors de la réaction.

10. Procédé suivant l'une des revendications précédentes 1 à 9, caractérisé en ce que l'on règle un rapport molaire réactif: agent de fluoration = (1 à 15):(10 à 1).

11. Procédé suivant l'une des revendications précédentes 1 à 10, caractérisé en ce que l'on règle un rapport molaire réactif: agent de fluoration = (1 à 7):(5 à 1).

12. Procédé suivant l'une des revendications précédentes 1 à 11, caractérisé en ce que l'on met en oeuvre, comme agent de fluoration, un mélange d'éthanes partiellement fluorés de formule générale

$$CF_{3-x}Cl_x\text{-}CH_{3-y}Cl_y$$

avec x = 0, 1, 2 et y = 0, 1, 2, 3.